# EUROPEAN PATENT APPLICATION

(11) **EP 4 459 362 A1**
(43) Date of publication of application: **06.11.2024**
(21) Application number: 21955297.3
(22) Date of filing: 27.12.2021
(51) Int. Cl.: G02C 7/04

(54) **OPTICAL LENS**

(71) Applicant: Pegavision Corporation, Taoyuan, Taiwan (TW)
(72) Inventor: HUANG, Yi-fang, Taoyuan, Taiwan (TW); LIN, Shih-siang, Taoyuan, Taiwan (TW)
(74) Representative: Winter, Brandl - Partnerschaft mbB
(86) International application number: PCT/CN2021/141528
(87) International publication number: WO 2023/122862

(57) **Abstract**

An optical lens includes an optical zone, and the optical zone includes a relax zone, a distance zone, and a defocus zone. The distance zone surrounds the relax zone. The defocus zone surrounds the distance zone and the relax zone. The diopter of the relax zone reduces along a direction from a center of the optical zone to an edge of the optical zone.

## Description

### BACKGROUND

### Field of Invention

The present invention relates to an optical lens.

### Description of Related Art

A unifocal design of a myopia correction lens can make a light focus at a center of the retina. However, axial length may be elongated continuously due to the image formed in back of the retina in the defocus zone. Therefore, although the unifocal lens can correct myopia, the unifocal lens will deepen the myopia.

In some designs for correcting myopia, such as a ring shape defocus design, may cause ghost images. When the myopia correction lens is used for children, the children may be unwilling to wear the lens and therefore the correction ability is poor. In addition, discomfort due to pressure may occur after long time accommodation for near vision.

Accordingly, it is still a development direction for the industry to provide an optical lens that can solve the problems above.

### SUMMARY

One aspect of the present disclosure is an optical lens applied in myopia correction.

In some embodiments, the optical lens includes an optical zone, and the optical zone includes a relax zone, a distance zone, and a defocus zone. The distance zone surrounds the relax zone. The defocus zone surrounds the distance zone and the relax zone. The diopter of the relax zone reduces along a direction from a center of the optical zone to an edge of the optical zone.

In some embodiments, an add power of the relax zone is in a range from +0.25D to +1.00D.

In some embodiments, a diopter of the distance zone is a constant value.

In some embodiments, a diopter of the distance zone increases along a direction from the relax zone to the edge of the optical zone.

In some embodiments, a diopter of the defocus zone increases along a direction from the distance zone to the edge of the optical zone.

In some embodiments, the optical zone further includes an enhance zone surrounding the defocus zone.

In some embodiments, a diopter of the enhance zone increases from an edge between the defocus zone and the enhance zone to the edge of the optical zone.

In some embodiments, a diopter of the enhance zone reduces from an edge between the defocus zone and the enhance zone to the edge of the optical zone.

In some embodiments, an absolute value of a diopter variation of the enhance zone is greater than an absolute value of a diopter variation of the defocus region, wherein the diopter variation is a ratio of the diopter divided by a lens radius.

In some embodiments, a diopter of the enhance zone is reciprocate in an interval.

In some embodiments, the optical lens is a hard contact lens or a soft contact lens.

In some embodiments, a material of the optical lens includes HEMA hydrogel or silicone hydrogel.

In some embodiments, the optical lens is configured to be stored in a lens preservation solution, the lens preservation solution includes a mydriatic agent with low concentration, and the mydricatic agent is configured to relax the ciliary muscle and to slow down the prescription increasing.

In some embodiments, the optical lens is an anti-blue light lens.

In some embodiments, the optical lens includes a cylinder power and a cylinder axis, and the optical lens is configured to correct astigmatism.

In the aforementioned embodiments, the eye discomfort caused by long time over- accommodation can be reduced by installing the relax zone. The diopter of the defocus zone increases along the direction from the distance zone to the edge of the optical zone, and is different from the diopter of the distance zone. As such, the problem of continuous elongation of the axial length due to the imaged formed in back of the retina in the defocus zone occurred in conventional unifocal lens can be improved to slow down the prescription increasing.

### BRIEF DESCRIPTION OF THE DRAWINGS

The invention can be more fully understood by reading the following detailed description of the embodiment, with reference made to the accompanying drawings as follows:
Fig. 1A is a top view of an optical lens according to one embodiment of the present disclosure;
Fig. 1B is a cross-sectional view taken along the line 1B-1B in Fig. 1A;
Fig. 2 is a diagram of a diopter and a radius of an optical lens according to one embodiment of the present disclosure;
Fig. 3 is a schematic of image formation of an optical lens according to one embodiment of the present disclosure;
Fig. 4 is a diagram of a diopter and a radius of an optical lens according to one embodiment of the present disclosure;
Fig. 5 is a diagram of a diopter and a radius of an optical lens according to one embodiment of the present disclosure;
Fig. 6 is a schematic of image formation of an optical lens according to one embodiment of the present disclosure;
Fig. 7 is a diagram of a diopter and a radius of an optical lens according to one embodiment of the present disclosure;
Fig. 8A is a top view of an astigmatic lens according to one embodiment of the present disclosure;
Fig. 8B is a top view of an astigmatic lens according to another embodiment of the present disclosure;
Fig. 9 is a diagram of diopter distribution of an astigmatic lens according to one embodiment of the present disclosure; and
Fig. 10 is a diagram of a diopter and a radius of an astigmatic lens taken along different angles in the optical zone according to one embodiment of the present disclosure.

### DETAILED DESCRIPTION

Reference will now be made in detail to the present embodiments of the invention, examples of which are illustrated in the accompanying drawings. Wherever possible, the same reference numbers are used in the drawings and the description to refer to the same or like parts.

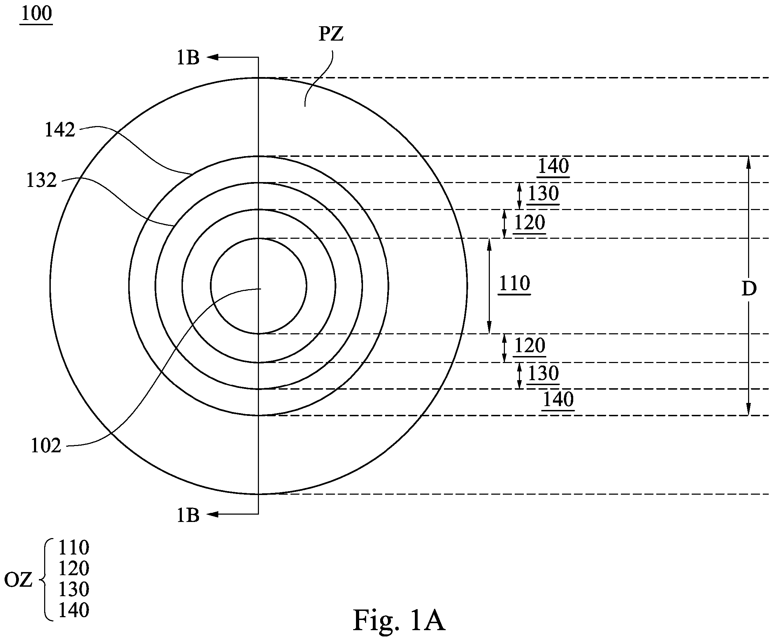

Fig. 1A is a top view of an optical lens 100 according to one embodiment of the present disclosure. The optical lens includes an optical zone OZ, a periphery zone PZ, and a center 102. The optical zone OZ includes a relax zone 110, a distance zone 120, a defocus zone 130, and an enhance zone 140. The distance zone 120 surrounds the relax zone 110, and the defocus zone 130 surrounds the distance zone 120 and the relax zone 110. The optical lens 100 of the present disclosure is applied in myopia correction and slowing down the prescription increasing. Low concentration mydriatic agent is added in a lens preservation solution of the optical lens 100 to avoid over-accommodation by relaxing the ciliary muscle and to slow down the prescription increasing. The optical lens 100 can be a hard contact lens, a soft contact lens, or a high oxygen permeability contact lens. A manufacturing method of a hard contact lens is performed by using an ultraprecision processing machine (e.g., Ametek Optoform80) to process a hard polymer material (e.g., PMMA) to form a combination of a front curve and a base curve having single or multiple radius of curvature. As such, the lens can satisfy the optical properties and is compatible with the corneal curvature. A manufacturing method of a soft contact lens is the cast molding method that using a back curve upper molding and a front curve bottom molding that having the shapes and optical properties of the lens. A liquid polymer material of the soft contact lens is filled in a chamber formed by the upper molding and the bottom molding. The material is heated to crosslink as a solid. The product is finished after hydration, packaging in preservation solution (e.g., PP blister packing), steriliztion, and labeling. The material of the optical lens 100 may include HEMA hydrogel or silicone hydrogel. The ingredient of hydrogel for soft contact lens includes Hydroxyethyl methacrylate (p-HEMA) such as Etafilcon A, of which the gas permeability is about 15-40 (Dk/t ×10⁻⁹). The silicone hydrogel is hydrogel containing silicon with high oxygen permeability such as senofilcon A, of which the gas permeability is about 50-150 (Dk/t ×10⁻⁹). In some embodiments, the optical lens 100 is an anti-blue lens. The anti-blue lens is formed by materials that can partially or completely absorb or block the blue light of 380nm to 500nm. The ability of absorbing the blue light can be achieved by common techniques such as dyeing or film plating.

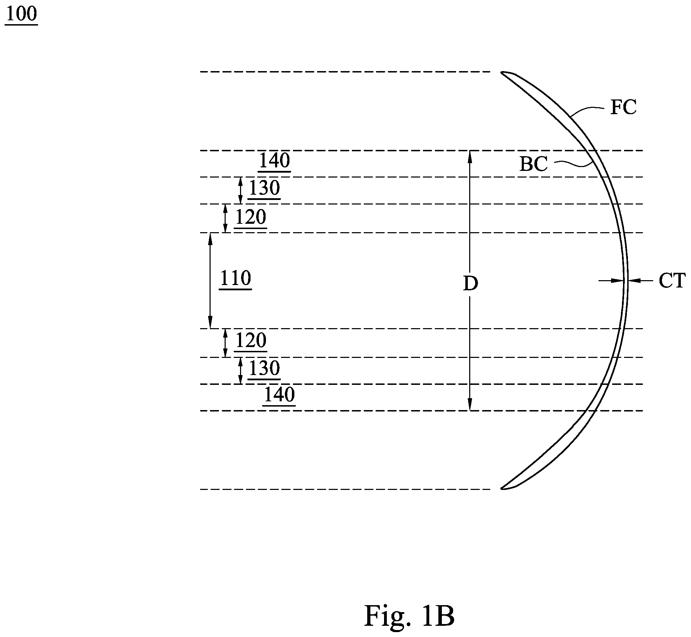

Fig. 1B is a cross-sectional view taken along the line 1B-1B in Fig. 1A. The optical lens 100 has a base curve BC, a front curve FC, a center thickness CT, and a diameter D. As an example, the diameter D in the present embodiment is 8 millimeter, but the present disclosure is not limited thereto. The base curve BC can be adjusted based on the eye properties of users so as to accommodate requirements of different symptoms or ages. The diopter is controlled by the front curve FC.

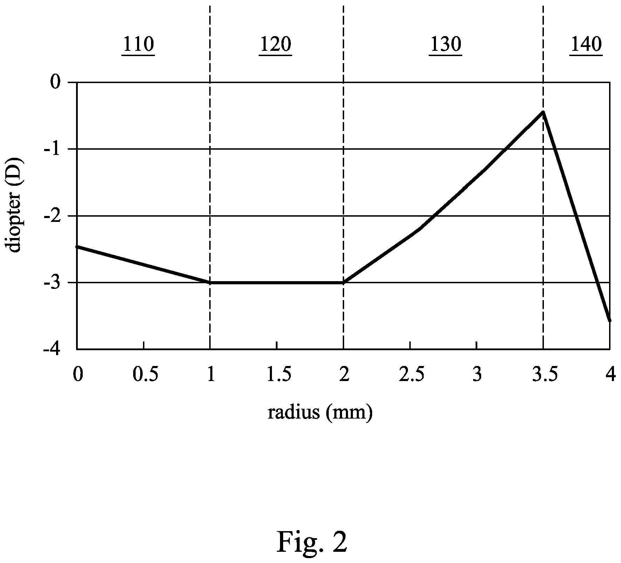

Fig. 2 is a diagram of a diopter and a radius of an optical lens according to one embodiment of the present disclosure. In the present embodiment, a range of the relax zone 110 is about from radius of 0 mm (the center 102) to radius of 1 mm. A range of the distance zone 120 is about from radius of 1 mm to radius of 2 mm. A range of the defocus zone 130 is about from radius of 2 mm to radius of 3.5 mm. A range of the enhance zone 140 is about from radius of 3.5 mm to radius of 4 mm. The ranges above are merely examples, and the present disclosure is not limited thereto.

As shown in Fig. 2, the diopter of the distance zone 120 is determined based on the prescription for myopia correction. In the present embodiment, the diopter of the distance zone 120 is -3.0D and is a constant value. The diopter of the relax zone 110 reduces along a direction from the center 102 of the optical zone OZ to an edge 142 of the optical zone OZ. That is, the diopter of the relax zone 110 is greater than the diopter of the distance zone 120. The add power of the relax zone 110 is in a range from +0.25D to +1.00D. In a proper embodiment, the add power of the relax zone 110 is in a range from +0.50D to +0.75D. For example, the diopter of the relax zone 110 of the present embodiment reduces from -2.5D to -3.0D. That is, the add power of the relax zone 110 is +0.5D. The eye discomfort caused by long time over-accommodation can be reduced by installing the relax zone 110.

The diopter of the defocus zone 130 increases along the direction from the distance zone 120 to the enhance zone 140 (that is, the direction towards the edge 142 of the optical zone OZ). In the present embodiment, the diopter of the defocus zone 130 increases from -3.0D to about -0.5D, and is different from the diopter of the distance zone 120. As such, the problem of continuous elongation of the axial length due to the imaged formed in back of the retina in the defocus zone 130 occurred in conventional unifocal lens can be improved.

In the present embodiment, the diopter of the enhance zone 140 reduces from the edge 132 between the defocus zone 130 and the enhance zone 140 to the edge 142 of the optical zone OZ. For example, the diopter of the enhance zone 140 reduces from -0.5D to -3.5D. As indicated by the line of the diopter value, a slope value corresponding to the diopter of the enhance zone 140 is greater than a slope value corresponding to the diopter of the defocus zone 130, and the slopes mentioned above have opposite sign. The diopter variation is defined as a ratio of the diopter divided by a lens radius. The absolute value of the diopter variation of the enhance zone 140 is 3.0D over 0.5, which equals 6. The absolute value of the diopter variation of the defocus zone 130 is 2.5D over 1.5, which equals 1.7. Accordingly, the absolute value of the diopter variation of the enhance zone 140 is greater than the absolute value of the diopter variation of the defocus zone 130. Through such difference between the diopter variations of the defocus zone 130 and the enhance zone 140, image disturbance can be eliminated. As such, image focusing in the distance zone 120 and the defocus zone 130 is improved, and the ability of myopia correction and defocusing are improved.

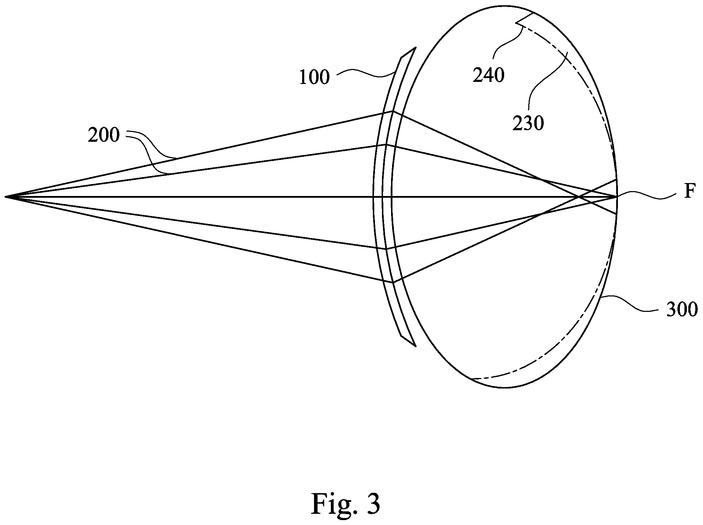

Fig. 3 is a schematic of image formation of an optical lens based on Fig. 2. A light 200 focuses on the focus F of the retina 300 after passing through the optical lens 100. The virtual image 230 formed in the defocus zone 130 and the virtual image 240 formed in the enhance zone 140 are indicated by dashed lines. As described above, since the virtual image 230 formed in front of the retina 300, myopia deepening due to continuous elongation of the axial length can be prevented. The virtual image 240 formed in the enhance zone 140 is more difficult to be recognized by human brain, and therefore there is an effect of shielding the enhance zone 140. In addition, since the slopes corresponding to the diopter of the enhance zone 140 and the diopter of the defocus zone 130 have opposite signs, the virtual image 240 is not connected with the image formed in the defocus zone 130 or even the virtual image 240 cannot be formed. As a result, the human brain can hardly recognized or process the virtual image 240 in the enhance zone 140, and therefore such design can enhance focusing of the images in the defocus zone 130 and the distance zone 120. As a result, the optical lens 100 of the present disclosure can provide clear near vision and far vision for users having myopia.

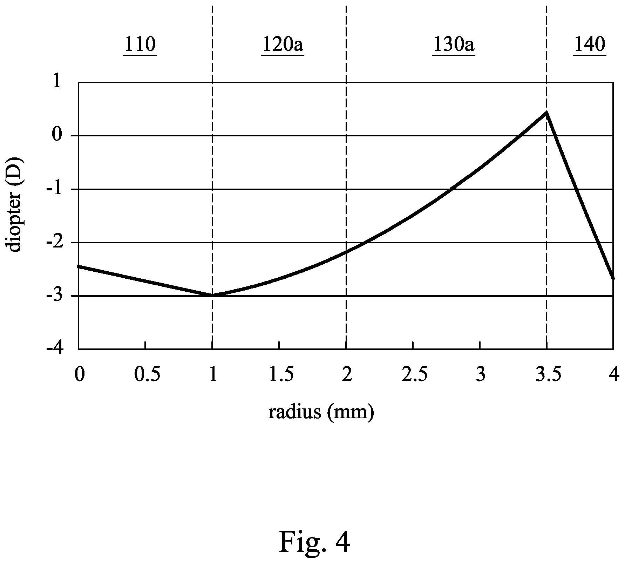

Fig. 4 is a diagram of a diopter and a radius of an optical lens according to one embodiment of the present disclosure. The present embodiment is substantially the same as the embodiment shown in Fig. 2, and the difference is that the diopter of the defocus zone 130a increases from the distance zone 120a to the defocus zone 130a. In other words, the diopter variation between the distance zone 120a and the defocus zone 130a is gradual. That is, the diopter variation is smoother. As such, a blur or a ghost image caused by obvious diopter variation can be reduced and a clear vision can be provided for the users.

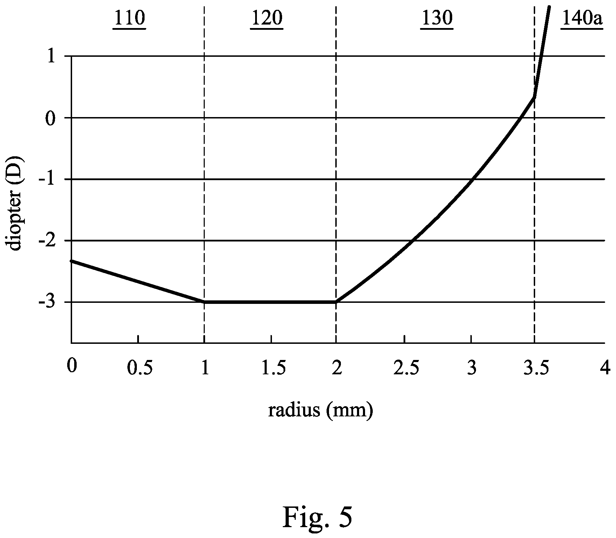

Fig. 5 is a diagram of a diopter and a radius of an optical lens according to one embodiment of the present disclosure. The present embodiment is substantially the same as the embodiment shown in Fig. 2, and the difference is that the diopter of the enhance zone 140a increases along a direction from the defocus zone 130 to the edge 142 of the optical zone OZ. The slopes corresponding to the diopter of the enhance zone 140 and the diopter of the defocus zone 130 have the same sign, and the slope corresponding to the diopter of the enhance zone 140 is greater than the slope corresponding to the diopter of the defocus zone 130 obviously.

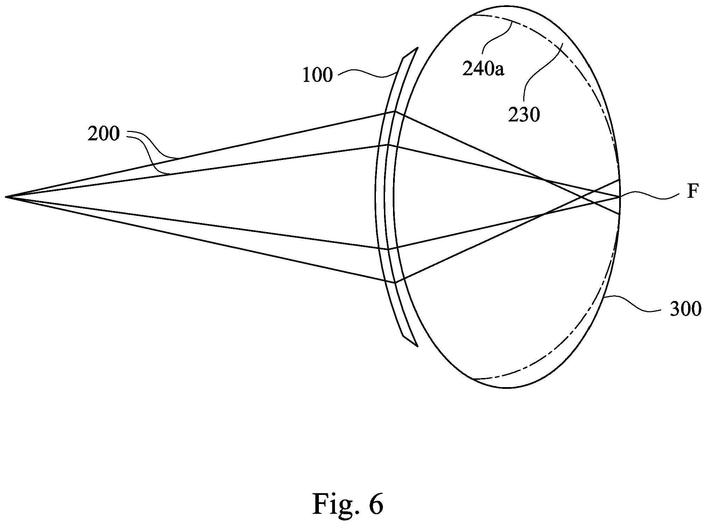

Fig. 6 is a schematic of image formation of the optical lens in Fig. 5. The virtual image 230 formed in the defocus zone 130 and the virtual image 240a formed in the enhance zone 140a are indicated by dashed lines. As described above, the human brain can hardly recognize or process the virtual image 240a in the enhance zone 140, and therefore such design can enhance focusing of the images in the defocus zone 130 and the distance zone 120. As a result, with such difference between the diopter variations of the defocus zone 130 and the enhance zone 140a, image disturbance can be eliminated to improve image focusing in the distance zone 120 and the defocus zone 130, and the ability of myopia correction and defocusing are improved.

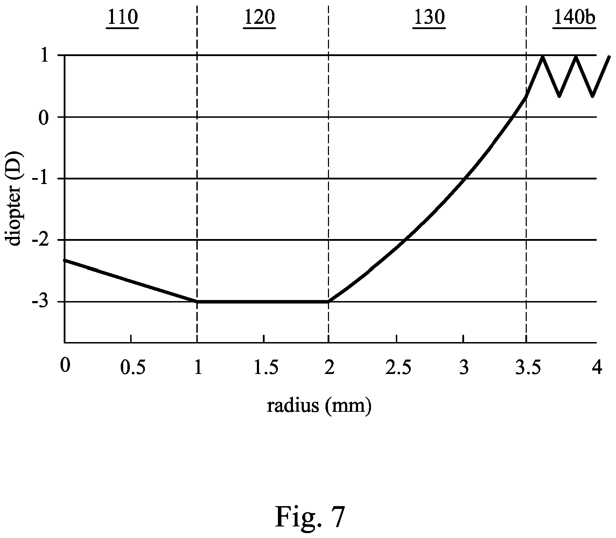

Fig. 7 is a diagram of a diopter and a radius of an optical lens according to one embodiment of the present disclosure. The present embodiment is substantially the same as the embodiment shown in Fig. 2, and the difference is that the diopter of the enhance zone 140b is reciprocate in an interval. For example, the diopter of the enhance zone 140 increases and decreases repeatedly in an interval from 1.0D to 4.0D. Therefore, such quick variation of the diopter in the enhance zone 140 make it difficult to form image, and the human brain can hardly recognize or process the virtual image in the enhance zone 140b. As such, with such difference between the diopter variations of the defocus zone 130 and the enhance zone 140b, image focusing in the defocus zone 130 and the distance zone 120 is improved and the ability of myopia correction and defocusing are improved.

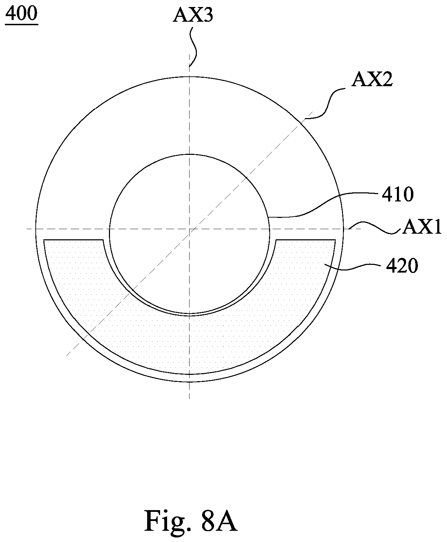

Fig. 8A is a top view of an astigmatic lens 400 according to one embodiment of the present disclosure. The astigmatic lens 400 includes an astigmatism optical zone 410 and an astigmatism stability thick zone 420. The astigmatism stability thick zone 420 of the present disclosure is positioned at the bottom of the lens. Such design is the prism-ballast type astigmatic lens 400.

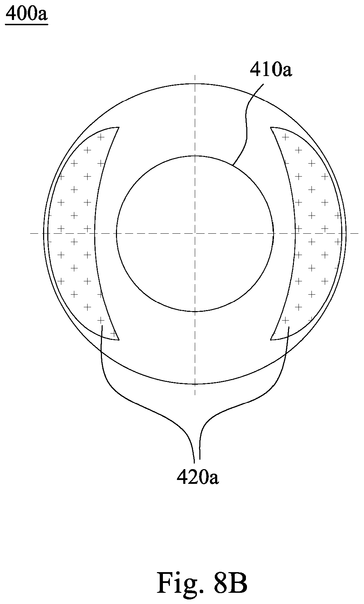

Fig. 8B is a top view of an astigmatic lens 400a according to another embodiment of the present disclosure. The astigmatic lens 400a includes an astigmatism optical zone 410a and an astigmatism stability thick zone 420a. Two astigmatism stability thick zones 420a are present respectively at the left hand side and the right hand side of the lens. Such design is the double slab-off type astigmatic lens 400a.

The astigmatism optical zones 410, 410a includes the relax zone 110, the distance zone 120, the defocus zone 130, and the enhance zone 140 aforementioned. That is, the astigmatism optical zones 410, 410a have the ability of myopia correction, pressure relief, and focusing improvement as described in the embodiments above. The astigmatism stability thick zones 420, 420a are configured to keep lens from rotation after the lens is worn by the users so as to maintain the correction function. The stability thick zones are not limited by those types as described above.

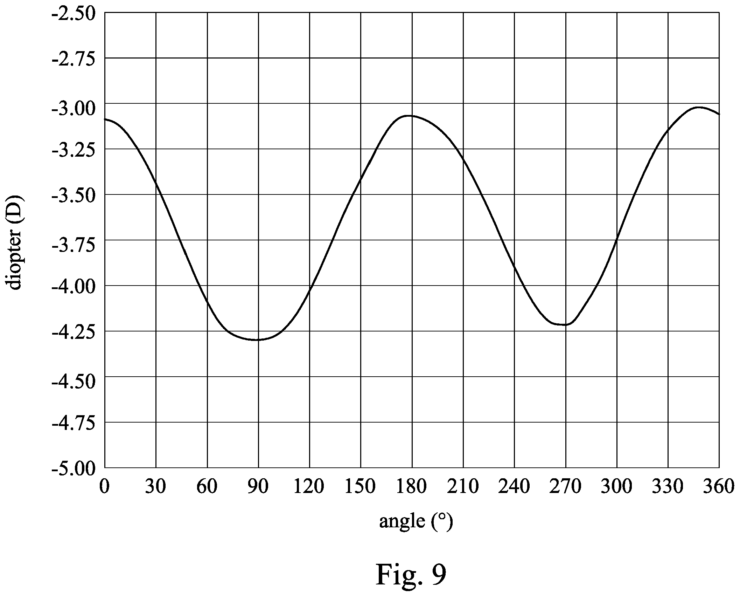

Fig. 9 is a diagram of diopter distribution of an astigmatic lens according to one embodiment of the present disclosure. Specifically, the astigmatism optical system includes double power magnitude (toric optics) such as spherical power, cylinder power, and cylinder axis. The embodiment shown in Fig. 9 includes a spherical power of -3.00D, a cylinder power of -1.25D, and a cylinder axis of 180 degrees. Therefore, the diopter at the positions corresponding to axis 0 degree and 180 degrees are about -3.00D, and the diopter at the positions corresponding to axis 90 degrees and axis 270 degrees are about -4.25D. The diopter variation of the relax zone 110, the distance zone 120, the defocus zone 130, and the enhance zone 140 described above are included herein, and the astigmatism optical property mentioned above are demonstrated by using the axis change in a distance zone as an example.

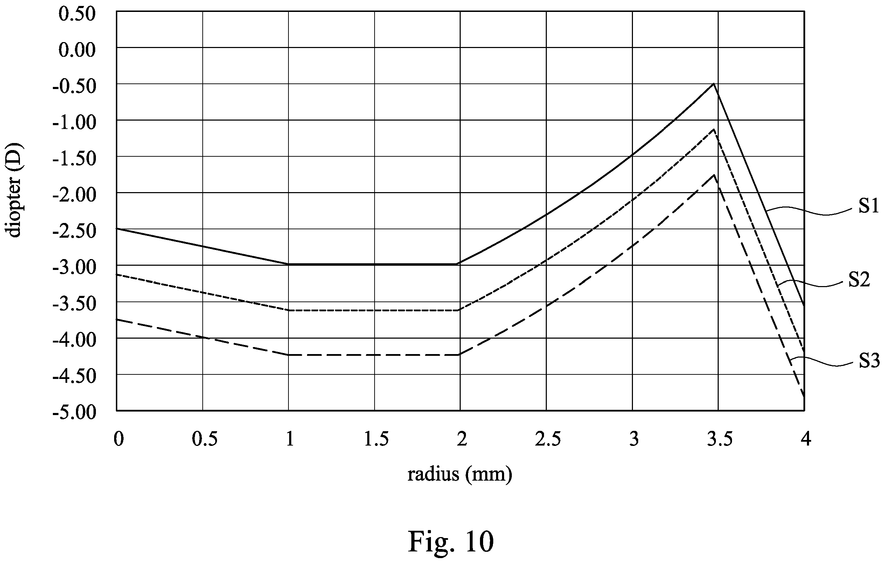

Fig. 10 is a diagram of a diopter and a radius of an astigmatic lens taken along different angles in the optical zone according to one embodiment of the present disclosure. Reference is made to Fig. 8A and Fig. 10. An axis AX1 of 0 degree, an axis AX2 of 45 degrees, and an axis AX3 of 90 degrees are illustrated in Fig. 8A. Curves S1, S2, and S3 are relation curves of the diopters and the radii along the axis AX1, AX2, and AX3.

The diopter distribution of the optical zone OZ shown in Fig. 3 is demonstrated herein. In the present embodiment, the spherical power is -3.00D, the cylinder power is -1.25D, the axis is 180 degrees, and the add power is +0.50D (that is the add power of the relax zone). The region from the radius of 1 mm to the radius of 2 mm is the distance zone, and the diopter of -3.0D is the prescription required for myopia correction.

Reference is made to Fig. 8A and Fig. 10. As shown by the curve S1, the relation between the diopter and the radius along the axis AX1 is substantially the same as the relation shown in Fig. 2. As shown by the curve S2, the relation between the diopter and the radius along the axis AX2 has a cylinder power of -0.63D releative to the curve S1. However, the curve S2 and the curve S1 have similar variation trend along the radius. As shown by the curve S3, the relation between the diopter and the radius along the axis AX3 has a cylinder power of -1.25D relative to the curve S1. However, the curve S3 and the curve S1 have similar variation trend along the radius. The advantages of pressure relief, myopia correction, and the astigmatism correction can be achieved by presenting aforementioned optical designs at the same side (front curve FC or the base curve BC) of the lens or be presented respectively at one of the sides of the lens. In some embodiments, the spherical power range of the astigmatic lens in a range from +10.0D to -10.0D, the cylinder power range is in a range from -0.50D to -3.50D, and cylinder axis is in a range from 5 degrees to 180 degrees.

Accordingly, the astigmatism lens can satisfy the requirements of the relation between the diopter and the radius, the cylinder power, and the cylinder axis as shown in Fig. 2. As such, such design can provide clear near vision and far vision for users having astigmatism. And such design has the advantages of the relax zone 110, the distance zone 120, the defocus zone 130, and the enhance zone 140 mentioned above.

In summary, the eye discomfort caused by long time over-accommodation can be reduced by installing the relax zone. The diopter of the defocus zone increases along the direction from the distance zone to the edge of the optical zone, and is different from the diopter of the distance zone. As such, the problem of continuous elongation of the axial length due to the image formed in back of the retina in the defocus zone occurred in conventional unifocal lens can be improved. As a result, with obvious diopter variations between the defocus zone and the enhance zone, image disturbance can be eliminated to improve image focusing in the distance zone and the defocus zone, and the ability of myopia correction and defocusing are improved.

Although the present invention has been described in considerable detail with reference to certain embodiments thereof, other embodiments are possible. Therefore, the spirit and scope of the appended claims should not be limited to the description of the embodiments contained herein. It will be apparent to those skilled in the art that various modifications and variations can be made to the structure of the present invention without departing from the scope or spirit of the invention. In view of the foregoing, it is intended that the present invention cover modifications and variations of this invention provided they fall within the scope of the following claims.

## Claims

1. An optical lens, applied in myopia correction and slowing down the prescription increasing, wherein the relax lens includes an optical zone, and the optical zone comprises:
a relax zone;
a distance zone surrounding the relax zone; and
a defocus zone surrounding the distance zone and the relax zone, wherein a diopter of the relax zone reduces along a direction from a center of the optical zone to an edge of the optical zone.

2. The optical lens of claim 1, wherein an add power of the relax zone is in a range from +0.25D to +1.00D.

3. The optical lens of claim 1, wherein a diopter of the distance zone is a constant value.

4. The optical lens of claim 1, wherein a diopter of the distance zone increases along a direction from the relax zone to the edge of the optical zone.

5. The optical lens of claim 1, wherein a diopter of the defocus zone increases along a direction from the distance zone to the edge of the optical zone.

6. The optical lens of claim 5, wherein the optical zone further includes an enhance zone surrounding the defocus zone.

7. The optical lens of claim 6, wherein a diopter of the enhance zone increases along a direction from an edge between the defocus zone and the enhance zone to the edge of the optical zone.

8. The optical lens of claim 6, wherein a diopter of the enhance zone reduces from an edge between the defocus zone and the enhance zone to the edge of the optical zone.

9. The optical lens of claim 6, wherein an absolute value of a diopter variation of the enhance zone is greater than an absolute value of a diopter variation of the defocus zone, wherein the diopter variation is a ratio of the diopter divided by a lens radius.

10. The optical lens of claim 6, wherein a diopter of the enhance zone is reciprocate in an interval.

11. The optical lens of claim 1, wherein the optical lens is a hard contact lens or a soft contact lens.

12. The optical lens of claim 1, wherein a material of the optical lens includes HEMA hydrogel or silicone hydrogel.

13. The optical lens of claim 12, wherein the optical lens is configured to be stored in a lens preservation solution, the lens preservation solution includes a mydriatic agent with low concentration, and the mydricatic agent is configured to relax a ciliary muscle and to slow down the prescription increasing.

14. The optical lens of claim 1, wherein the optical lens is an anti-blue light lens.

15. The optical lens of claim 1, wherein the optical lens includes a cylinder power and a cylinder axis, and the optical lens is configured to correct astigmatism.
